# EUROPEAN PATENT APPLICATION

(11) **EP 2 587 252 A1**
(43) Date of publication of application: **01.05.2013**
(21) Application number: 11186650.5
(22) Date of filing: 26.10.2011
(51) Int. Cl.: G01N 21/55, G01N 33/543, B82Y 15/00, B82Y 30/00, G01N 15/14

(54) **Individual molecule detection by plasmonic nanoparticles**

(71) Applicant: Sönnichsen, Carsten, 55128 Mainz (DE)
(72) Inventor: Sönnichsen, Carsten, 55128 Mainz (DE); Ament, Irene, 55128 Mainz (DE)
(74) Representative: Müller Schupfner & Partner

(57) **Abstract**

The invention relates to an apparatus and a method for sensing the presence and/or the behaviour of individual target molecules in a sample, the apparatus comprising a plasmonic nanoparticle (6) which is exposed to the sample; a light source (1) illuminating the plasmonic nanoparticle (6) in a dark-field geometry; and an optical device (10, 11, 12) for gathering and spectrally analysing the light scattered from the nanoparticle (6). The light source is a white light laser (1). This set-up allows the detection of individual binding and unbinding events of target molecules to the nanoparticle.

## Description

### Field of the Invention

The invention relates to a method and apparatus for sensing the presence and/or the behaviour of individual target molecules in a sample by using plasmonic nanoparticles.

Plasmonic nanoparticles react to refractive index changes in their direct environment by a shift of the plasmon resonance frequency. This can be monitored on single nanoparticles or ensembles of nanoparticles by optical spectroscopy, as described in the review article "Localized Surface Plasmon Resonance Sensors" by K. M. Mayer and J. H. Hafner, Chem. Rev. 2011, 111, 3828-3857. Although it is possible to study a single nanoparticle, signal to noise ratio and time resolution was so far not sufficient to identify individual molecular binding events on that nanoparticle.

However, there is a need to study the complex conformational dynamics, molecular interactions and spatial diffusion of individual dissolved molecules. Other experimental techniques which provide access to molecular events in solution commonly require fluorescent dye labels attached to the molecule of interest. However, such analyte modification could potentially influence biological processes. Thus, it is an object of the invention to find a method and apparatus for detecting individual unlabeled biological molecules, in particular proteins.

This object is achieved by the apparatus of claim 1 and the method of claim 7. Preferred embodiments are described in the dependent claims.

### Description of the Invention

The invention has provided a method and apparatus for sensing the presence and/or the behaviour of individual target molecules dissolved in a sample, wherein single nanoparticles can be used to detect individual unlabeled target molecules, in particular proteins, with high temporal resolution. This is achieved through the detection of a slight shift in the plasmon resonance frequency, in particular when an individual target molecule binds or detaches from the nanoparticle. Such individual binding events can be observed with the apparatus of the invention with enhanced sensitivity.

Generally, the measured shift in the plasmon resonance frequency can indicate the presence of a target molecule in the proximity of the nanoparticle, wherein the shift may be caused by a binding/unbinding event of one individual target molecule to the nanoparticle - wherein the target molecule may bind either directly to the nanoparticle, or to a coating or a capture molecule attached to the nanoparticle. Each of these alternatives will be referred to in the following as "bound to the nanoparticle", whether the target molecule is bound directly to the nanoparticle or to a coating or capture molecule on the nanoparticle. The order of magnitude and/or the time evolution of the shift in the plasmon resonance frequency, which can be recorded with unprecedented resolution by means of the invention, allows to study the behaviour of an individual target molecule bound or in close proximity to the nanoparticle. The "behaviour" can be for example a conformational change in a target molecule, such as a folding or denaturing process of a protein, or the attachment of another molecule to the target molecule, or the fluctuations during equilibrium coverage of the nanoparticle.

In comparison with previously described techniques, the signal to noise ratio is significantly improved by at least 4-6 orders of magnitude and allows the direct identification of individual molecular binding and unbinding events. Further, the method enables the study of protein folding dynamics, protein adsorption processes and kinetics, as well as non-equilibrium soft matter dynamics on the single molecule level, whereas simulations predict the ability of the sensor concept to detect the presence of small molecules below a molecular weight of one kDa. These improvements are possible through the use of an intense light source, namely a white light laser, in the apparatus and method of the invention, preferably having a power output of > 2W. The stronger light source allows using smaller nanoparticles, which have a sensing volume tailored to the molecular size, which increases the perturbation and therefore the shift in resonance frequency caused by an individual molecule.

To achieve contrast, the white light laser illuminates the nanoparticle preferably in a dark-field geometry, such that only light scattered by a nanoparticle is directed to and picked up by the optical device/detector. The concept of optical dark-field spectroscopy is described e.g. in "Drastic reduction of plasmon damping in gold nanorods" by Sönnichsen, C. et al., Phys. Rev. Let. 88, 077402 (2002).

In one embodiment, the dark-field illumination is achieved by a prism that is illuminated from the side such that a total internal reflection at a surface of the prism, or the surface of a glass capillary containing the sample and the nanoparticle, occurs, thereby preventing illumination light from reaching the detector. The nanoparticle is preferably disposed on the other side of said surface, within the realm of the evanescent light wave. The prism is preferably a half-cylinder, thereby allowing adjustment of the angle of incidence to the refractive index of the sample, and is preferably made of glass.

According to another embodiment, the dark-field contrast is created through the use of a dark-field condenser. In this embodiment of the apparatus, the illumination does not necessary produce a total internal reflection. The necessary contrast is generated by illuminating under large angles and collecting light only at smaller angles than the smallest illumination angle.

Other ways of recording the plasmon resonance spectra can also be employed.

The scattered light is preferably gathered in an optical microscope objective and analysed by a spectrometer which is coupled to a detector, preferably a CCD (charge-coupled device) camera, which detects and/or records the spectrum generated by the spectrometer. The exposure time per recorded spectrum is for example between 1 ms and 100ms.

In a preferred embodiment, an intensified or amplified CCD camera is used as a detector, in particular an electron-multiplying CCD (EMCCD) camera, which further increases the sensitivity and the time resolution, thereby allowing the dynamics of individual molecule binding events to be studied. Preferably the spectra and therefore the plasmon resonance frequency can be recorded at a time resolution of between 50µs and 500ms, preferably between 1-100ms, most preferred between 10-50ms. In addition, a signal amplification can be switched on.

Preferably, the apparatus of the invention also comprises a processing device, such as a computer or other calculation machine, for evaluating the recorded spectra. For example, the spectral data may be pre-processed in order to suppress random noise, e.g. by smoothing. Preferably, a suitable curve (e.g. Parabola or Lorentzian) is fitted to the recorded spectra to find the peak, i.e. the plasmon resonance frequency, with the highest possible accuracy. The processing device may also comprise a digital storage medium, e.g. a hard disc, optical disc etc., for storing the spectral information.

The sample containing the target molecules is preferably a liquid such as water or saline solution, and is e.g. brought into contact with the nanoparticle in a micro-capillary, in which the nanoparticle is immobilized, allowing liquid exchange by pumping the liquid sample through the capillary. The refractive index between the micro-capillary and the prism surface or dark-field condenser is preferably matched with immersion oil.

The target molecules are preferably biological molecules (e.g. proteins) with a size of 6-100nm, preferably 10-50nm, wherein the size corresponds to the diameter in the longest dimension. In particular, the target molecules may be polypeptides or proteins having a molar weight between 1 and 3.000 kDa, preferably between 50 kDa and 1000 kDa. For smaller proteins, with molar weights e.g. between 200 and 1000 kDa, especially the binding kinetics can be investigated, for larger proteins with molar weights above about 500 or 1000 kDa, the inventive apparatus and method can additionally be used to study conformational changes and/or folding or denaturising processes of the protein. The inventive apparatus has been tested on fibronectin, which has a molar weight of 450 kDa and a globular shape of size of about 12 nm. Preferably, the target molecules are unlabelled, i.e. do not contain a dye, radioactive or other label attached to the biological molecule.

The nanoparticles are preferably of gold or silver, and preferably have the shape of sphere-capped cylinders. The aspect ratio is preferably above 2, more preferred between 2.5 and 4.5, most preferred between 3 and 4, and the width is preferably between 20 and 45 nm. As described below, simulation experiments allow to tailor the size and shape of the nanoparticle to the desired target molecule. For example, the nanoparticle preferably has a diameter of 1-6 times greater than the target molecule. Preferably, only a single nanoparticle is used, the geometry of which may be tailored to the target molecule, but there exist useful embodiments in which several nanoparticles are used in one experimental set-up.

Since the apparatus and method of the invention are suitable for monitoring binding and unbinding of target molecules to the surface of the nanoparticle, the plasmonic nanoparticle surface may be chemically modified (functionalized), or coated with a certain substance or membrane, to specifically attract a given target molecule. For example, the surface may be coated with certain "capture" molecules, which interact specifically with the target molecules. This allows to study the binding and unbinding constants and kinetics of the target molecules with regard to a specific binding partner. The capture molecules can be conjugated to the nanoparticle e.g. by means of a self-assembled monolayer (SAM) formation on the nanoparticle surface and standard bioconjugate linkers. For example, the capture molecules can be antigens which are conjugated to the nanoparticle surface and which bind specifically to certain antibodies. Alternatively, the capture molecules can be biotin, which binds to streptavidin. The biotin-streptavidin interaction has already been used for biological assays on the basis of Localised Surface Plasmon Resonance, as described in the review article by K.M. Mayer and J.H. Hafner (see above), but the sensitivity so far has been ranging from picolmolar to nanomolar concentrations, whereas the invention provides a single-molecule sensitivity. Other biomolecular interactions which can be studied with the apparatus and method of the invention include nucleic acid hybridization, protein-carbohydrate, cytochrome-inhibitor, aptamer-protein, and toxin-receptor interactions. For the first time, it is possible to study the behaviour (e.g. binding, unbinding, folding, binding kinetics including kinetic constants, etc.) of single target molecules in these interactions by means of plasmonic nanoparticles.

Such nanoparticles - coated or uncoated - can be inserted into living cells (see "Noble Metals on the Nanoscale" by Jain, P. K., Huang, X., EI-Sayed, I. H. & El-Sayed, M. A. Optical and Photothermal Properties and Some Applications in Imaging, Sensing, Biology, and Medicine. Acc. Chem. Res. 41, 1578-1586 (2008)) and examined *in vivo* by the inventive apparatus and method.

Another preferred way to improve the sensitivity is an extremely stable set-up of the apparatus, for example by placing it on a vibration-damped optical table.

The apparatus is preferably adapted to carry out the method of the invention, as further described below.

The invention is also directed to a method of sensing the presence and/or the behaviour of individual target molecules in a sample, comprising the steps of exposing a plasmonic nanoparticle (6) to the sample containing the target molecules; illuminating the plasmonic nanoparticle (6) by means of dark-field illumination; gathering the light scattered from the nanoparticle, spectrally investigating it and analysing the spectrum to detect a shift in the plasmon resonance frequency, wherein the light source used for the illumination is a white light laser.

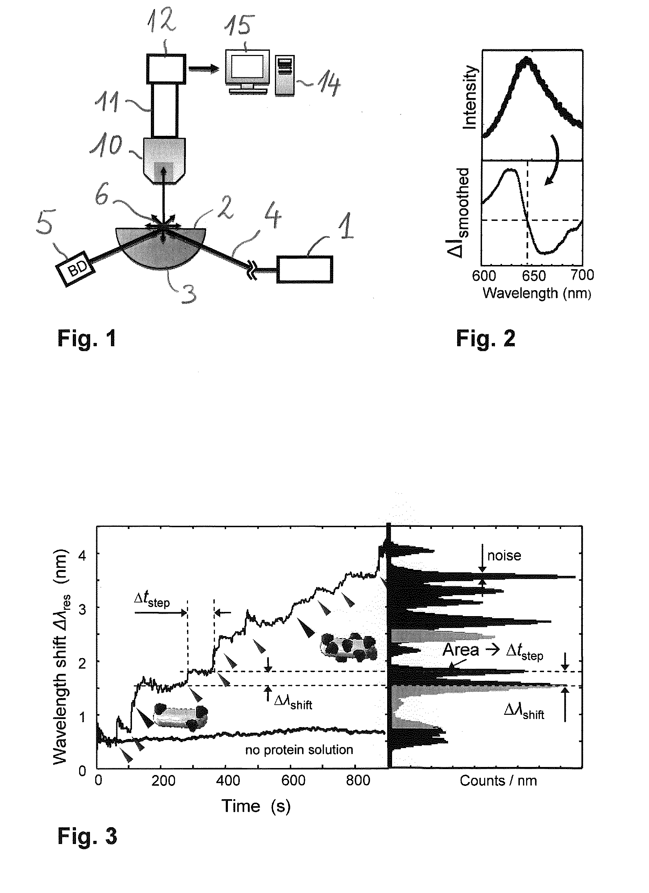

Molecular adsorption processes induce only very small shifts in the plasmon resonance when compared to the plasmon linewidth of about 50 nm. However, according to a preferred embodiment of the invention, the plasmon resonance wavelength can be determined with an accuracy of 0.003-0.2 nm, preferably 0.01-0.05 nm, most preferred about 0.03 nm by fitting the entire spectral resonance, as shown in Fig 2 (upper). The mathematical function used for the fitting may be a Gaussian, Parabola or Lorentzian fit.

In the inventive method, the resonance spectrum can be recorded at a time resolution of between 50µs and 500ms, preferably between 1-100ms, most preferred between 10-50ms. This allows to monitor the kinetics of the shift in resonance frequency, allowing investigation of binding kinetics and conformational changes in the target molecule.

Preferably, the shift in plasmon resonance frequency during one binding event, recorded at the above-mentioned time resolution, and averaged over several binding events, is fitted with a curve or mathematical function allowing determination of the time scale of the binding event. For the binding of approximately globular biological molecules, the curve to be fitted is Δλ_{shift} = A (1-e^{-t/T}), which gives a time scale T of about 1-2s for fibronectin (see experiments below).

According to a preferred embodiment, a shift in plasmon resonance frequency, recorded at the above-mentioned time resolution, allows determination of a conformational change in the target molecule while it is bound or in close proximity to the nanoparticle, thereby allowing the study of e.g. folding and denaturising processes in proteins.

### Brief description of the drawings

The invention will now be described by means of preferred embodiments with reference to the attached drawings, in which:
Fig. 1 is a schematic view of the apparatus according to an embodiment of the invention;
Fig. 2 is a graph of the detected spectrum of light scatted from a nanoparticle, before and after adsorption of a target molecule (above), and the difference in light intensity between the two curves (below):
Fig. 3 shows the resonance wavelength of an individual nanoparticle during individual protein attachment events (steps) and without protein solution (left) and a histogram of the same time trace showing distinct peaks for each step;
Fig. 4 is a plot of shift in resonance frequency over time showing the mean adsorption behaviour averaged from 8 individual adsorption events (not shown) taken from a single nanoparticle. The deviation from a step-like form suggests a protein denaturing in the timescale of 1-2 s (inset).
Fig. 5 shows that the fluctuations in the resonance frequency of a nanoparticle in pure water (0.04 nm standard deviation) is significantly lower that the fluctuation amplitude of 0.11 nm in a protein surfactant mixture (25µg/ml fibronectin, 0.2wt% SDS) due to equilibrium protein coverage fluctuations;
Fig. 6 shows a schematic representation of three different-size nanoparticles and their respective sensing volumes;
Fig. 7 shows a graph of cumulative probability of measured resonance shifts per adsorbed molecule within one trace for large rods (circles, mean shift 0.17nm), medium rods (squares, mean shift 0.30nm), and small rods (triangles, mean shift 0.51 nm). The respective noise level is shown on the left hand side;
Fig. 8 shows a simulated shift induced by a 12 nm diameter adsorbing protein compared to the noise level of the inventive apparatus. In the grey area, the signal to noise level is above one.

### Experiments

An embodiment of the inventive apparatus is schematically shown in Fig. 1, wherein the white light laser 1 is illuminating the surface 2 of a half-cylindrical prism 3 with a laser beam 4. The beam is reflected from the surface in total internal reflection and is finally absorbed by the beam deflector 5. The nanoparticle 6 is immobilised within a capillary (not shown) on the prism surface, and scatters light reaching it in the form of an evanescent wave on the opposing side of the reflection surface. About 10% of the scattered light is gathered by the objective 10, passed on to the spectrometer 11 to measure the light scattering spectrum, which is passed on to the EMCCD 12. The camera 12 is connected to a computer 14 for further analysis, which in turn is connected to a screen 15 for viewing the results.

In detail, a commercial upright microscope from Zeiss (Axioscope) equipped with a white light laser (WLL) from Koheras (SuperK-Power, spectral regime 460 nm - 2400 nm, total average power output 2.8 W) as the illumination source was used. The scattered light was analyzed by an ImSpector V8 (Specim) coupled to an EMCCD camera (iXon, Andor DV885). The exposure time for medium rods was 10 ms with a cycle time of 13.7 ms. The gain was adjusted if needed, normally ranging between no gain and gain factor five.

The sample was introduced by means of a flow cell, namely a home-built flow cell consisting of a glass capillary (2 mm x 0.1 mm) attached with glue to flexible tubing. Thus, a liquid exchange was easily possible. The refractive index between the flow cell and the glass surface was matched with immersion oil.

The rod-shaped gold nanoparticles (nanorods) which were used as sensors were prepared in a two step wet-chemical synthesis. In the first step seeds were grown from HAuCl4 solution. These seeds were then enlarged in a second step to gold nanorods. The dimensions used in the experiments range from small (14 nm x 40 nm, λᵣₑₛ = 674 nm) over medium (35 nm x 78 nm, λᵣₑₛ = 635 nm) to large (50 nm x 107 nm, λᵣₑₛ = 664 nm) rods. Particles were immobilized with 1M NaCl in the flow cell. Upon inspection in the TIR microscope, they were clearly visible to the eye as bright red spots on a dark background. Differences appeared in their intensity that scales quadratically with the particle volume. The immobilized gold nanorods were rinsed extensively in pure water and 10 mM filtered Phosphate buffered saline (PBS).

To investigate molecular binding events, the blood plasma protein fibronectin (molar weight 450 kDa) was used as target molecule and introduced into the capillary. To prepare the test samples, fibronectin from bovine plasma (F1141-1 mg), and sodium dodecyl sulfate (SDS) were bought from Sigma. Different concentrations of protein solution were prepared by diluting a concentrated fibronectin solution with PBS. The optimal concentration for the detection of individual protein adsorption steps was found to be 1.25 µg/ml.

Before starting the measurements, the apparatus was switched on and left for about one hour to reach thermal and mechanical equilibrium, thereby stabilising the wavelength. When a stable resonance wavelength was achieved for several minutes, one end of the flow cell tubing was put into the protein solution and gravitational force generated a flow towards the gold nanorod under investigation. At this point in time, the measurement was started and no further adjustments at the setup were carried out during measurement.

A shift in the plasmon resonance clearly indicates adsorption of fibronectin to the gold nanoparticles. The plasmon shift follows the Langmuir adsorption curves known from ensemble experiments (e.g. surface plasmon resonance sensors, SPR) with the characteristic time scaling with fibronectin concentration. When the fibronectin concentration is decreased down to 1.25 µg/ml, discrete steps start to appear in the resonance wavelength time trace (see Fig. 3). These discrete steps are caused by individual attaching molecules. The inventors found that using wavelength histograms as shown on the right of Fig. 3 is the best way to systematically identify individual protein binding steps. In the histogram, each Gaussian peak corresponds to a molecule attaching to the plasmon sensor. Neighboring Gaussian peaks are separated by Δλ_{shift} and their integrated area yields the step time Δ*t*ₛₜₑₚ. Assuming delta-like events, the width corresponds to the experimental noise level. For the experiment shown in Fig. 3, the average step height Δλ_{shift} is approximately 0.3 nm with about Δ*t*ₛₜₑₚ = 50 s between steps and a noise level of 0.07 nm.

It was carefully checked that the observed steps indeed correspond to individual protein binding events. The attachment frequency (the inverse of the time between steps Δ*t*ₛₜₑₚ) increases as expected with protein concentration and is in quantitative agreement with theoretical predictions assuming diffusion limited rates. To validate the step height Δλ_{shift}, it was checked if it varies with protein size. Fibronectin is a dimeric protein that can be cleaved into its monomeric constituents by tris(2-carboxyethyl) phosphine, TCEP. Indeed, the average shift or step height induced by monomeric fibronectin is about half the value as that of dimeric fibronectin.

As an additional validation, the desorption of the proteins was followed by washing with sodium dodecyl sulfate (SDS). Clearly pronounced desorption steps were observed. Taken together, the control experiments strongly confirm the achievement of single molecule sensitivity.

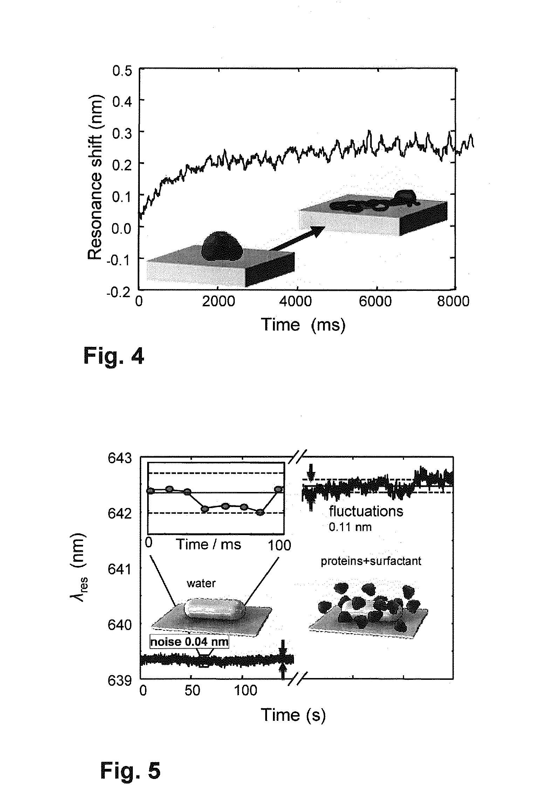

Being able to resolve single protein binding, it was attempted to time-resolve the adsorption process itself - information fundamentally hidden in ensemble measurements. For this, all steps, i.e. several shifts in plasmon frequency each occurring during one binding event, within one measurement were manually superimposed and averaged to increase the signal to noise ratio further. As shown in Fig. 4, the inventors observed a plasmonic red-shift during the adsorption process with about 1.95 s timescale, when the time trace was fitted to Δλ_{shift} = A (1-e^{-t/T}), where A describes the height of the final shift, and T is the time scale. Please note that Fig 4 shows an average curve of 8 adsorption events taken from a single nanorod. Similar observations show up on superimposed steps of other measurements confirming the existence of a real physical process. It is believed that this slow red-shift within one adsorption event arises from a slow denaturing of the protein on the surface, bringing it closer to the particle (Fig. 4 inset). Such denaturing of proteins near metal surfaces is, by itself, a well known phenomenon (Anand, G., Sharma, S., Dutta, A. K., Kumar, S. K. & Belfort, G. Conformational Transitions of Adsorbed Proteins on Surfaces of Varying Polarity. Langmuir 26, 10803-10811 (2010).). The inventive method allows for observation of the temporal evolution of this process in detail and should trigger the development of models to explain the observed denaturing timescale.

The inventive apparatus and method can be used to study molecular fluctuations, in particular equilibrium coverage fluctuations, which give direct access to binding constants without mass transfer complications, see Lüthgens, E. & Janshoff, A. "Equilibrium coverage fluctuations: A new approach to quantify reversible adsorption of proteins" ChemPhysChem 6, 444-448 (2005). In addition, equilibrium fluctuations are linked to non-equilibrium properties via fluctuation-dissipation theorems, see Kubo, R. "Fluctuation-Dissipation Theorem" Rep. Prog. Phys. 29, 255-& (1966). The recent discovery of unexpected fluctuation theorems, e.g., the Jarzynski theorem, have increased the demand for experimental techniques to study molecular fluctuations, see Jarzynski, C. "Nonequilibrium equality for free energy differences" Phys. Rev. Let. 78, 2690-2693 (1997). In the present experiment, equilibrium coverage fluctuations of fibronectin was observed on the sensor surface under conditions of weak binding affinity. To adjust the binding affinity, both fibronectin and sodium dodecyl sulfate (SDS) were added and an increase in the fluctuation amplitude from 0.04 nm to 0.11 nm was observed, see Fig. 5.

### Simulation of expected shifts

In addition to the experiments, the expected shift per adsorbed molecule, Δλ_{shift}, was simulated. It was proportional to the change in refractive index, Δ*n*, the plasmonic sensitivity, S = dλ/d*n*, and the volume fraction V_{frac} of the protein Vₚᵣₒₜ to the total sensing volume *V*ₛₑₙₛ, i.e. V_{frac} = *V*ₚᵣₒₜ/*V*ₛₑₙₛ. Together it follows that Δλ_{shift} = S Δ*n V*_{frac}. All simulations were performed for gold nanorods with the aspect ratio of *AR* = 2.25, the dielectric function of gold and a spherically capped cylinder as shape for the gold nanorod.

The refractive index change induced by the protein was approximated with Δ*n* = 0.17 taking the literature value for a typical refractive index of a biomolecules of 1.55, which is 0.17 difference to water. The sensitivity S of these particles was numerically calculated. It changed only slightly from 160 nm/RIU to 240 nm/RIU for rods with diameters between 5 nm and 50 nm. Hence, the important term for the size of the induced shift per molecule Δλ_{shift} was the volume fraction *V*_{frac}, the ratio between the fixed analyte volume *V*ₚᵣₒₜ and the sensing volume *V*ₛₑₙₛ.

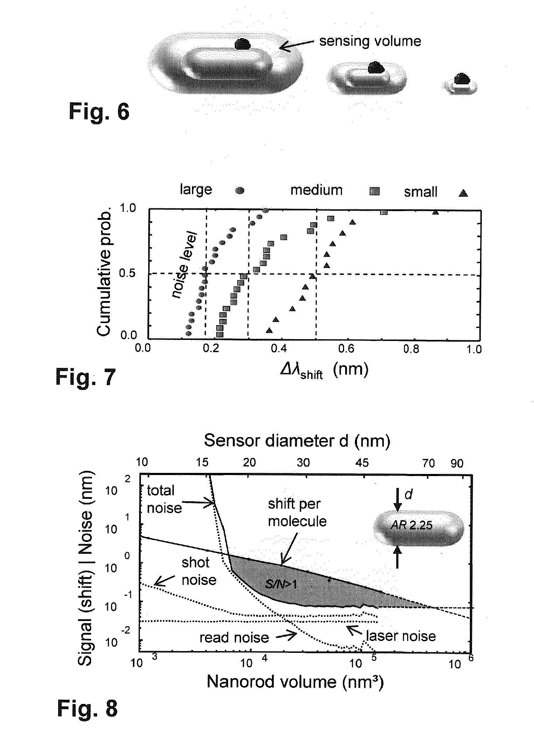

To estimate the volume fraction, the sensing distance d that produces 1/e of the maximum shift was determined first.(i.e. a gold nanorod in a medium with n = 1.5). Technically, the plasmon resonance was simulated for a given particle size with an increasing shell of protein around it using the boundary element method (BEM). From the sensing distance, the sensing volume *V*ₛₑₙₛ is obtained by π·*d [a·b + a·d + b·d + 4*/*3*·*d*²*]* with rod length a, width b and sensing distance d. It was found that the sensing volume is approximately equal to the particle volume, as illustrated in Fig. 6.

Multiplication of these parameters (change in refractive index Δ*n*, plasmonic sensitivity S, volume fraction *V*_{frac}) gives the shift per molecule, Δλ_{shift}.

### Simulation of the setup noise

To get insight into the final values for the noise from the participating noise sources, each of them was measured individually. We then included the power of the light source, the scattering efficiency of the sample, measured transmission losses in the light path, and the quantum efficiency of the EMCCD camera to determine the final noise values.

The laser noise was measured for 10000 frames (*t*ₑₓₚ=30 µs - 0.1 s) on a bright white silica particle (diameter 314 nm). The normalized standard deviation of the intensity at various wavelength gave values around 1.5%, which is in good agreement with manufacturer specification. The read noise is the noise introduced by the CCD camera while reading out, independent from the signal level. It was measured for several gain values by subtracting two dark pictures with minimal exposure times and extracting the standard deviation of the noise. As two pictures were used, the noise is reduced by square root of two. The gain dependent read noise for 20 binned pixels was measured to be 15.903 + (1.151·gain) + (0.007·gain²).

Due to the thermoelectric cooling of the camera, dark current noise is negligible. The shot noise originates from photon statistics and is given by the square root of the number of arriving photons.

To determine the noise specific to a given particle size, the scattering spectrum for a set exposure time of 10 ms was calculated using the measured effective setup transmission for the given illumination flux of 2.4·10²¹ photons per second, wavelength and m² for a particle scattering cross section of 3.9·10⁻¹⁵ m². The maximum possible gain without saturating the camera was used. Each wavelength of this 'perfect spectrum' was mathematically overlaid with random values for all noise terms taken from a Gaussian distribution multiplied with the previously determined noise.

The results of these noise calculations are plotted in Fig. 8, where the total noise level is compared with the calculated shift in plasmon resonance frequency expected at different nanoparticles volumes, wherein we find a signal-to-noise (S/N) level of greater than 1 for nanoparticles with widths between 20 - 45 nm

### Determination of optimal size of nanoparticle

To fully understand the future potential of single plasmonic nanoparticles as molecular sensors, the inventors investigated theoretically and experimentally the limits of the method, in particular the requirements needed for detecting smaller molecules.

In particular, the size and shape of the nanoparticle strongly influences the sensitivity of the method. It is not trivial to choose the optimal nanorod size, since there is a trade-off of signal strength and resonance accuracy. Larger nanorods generally give stronger light scattering signals and should allow to determine the plasmon resonance with higher accuracy. On the other hand, the sensing volume that influences the resonance position also increases with particle size (see simulation results above and Fig. 6) which decreases the perturbation caused by a single molecule. Indeed, experiments using large, medium and small gold nanorods (14 nm, 35 nm, 50 nm width) show larger median plasmon shifts for smaller particles (Fig. 7). Using experimentally determined noise levels from the laser and camera and the sensor response (shift per molecule) calculated with the boundary element method, the signal to noise ratio for rods between 10 and 50 nm in width (aspect ratio AR 2.25) was studied. The results show an optimal size region with signal to noise S/N > 1 for particles with widths between 20 - 45 nm (Fig. 8) for the noise levels of the current apparatus. By reducing the electronic or laser noise even further, even smaller particles can be used.

The theoretical description allows to determine the limit of plasmonic nanoparticle sensors to detect small molecules. Increasing the laser power up to a point where the absorbed light increases the particle temperature by 1 K, it is possible to detect molecules of more than 4 nm diameter within the current geometry and setup using nanorods with 20 nm width. Smaller nanorods improve the detection limit even more but require stronger lasers. Further improvements could be brought about by the use of plasmonic structures with even higher sensitivity such as metamaterials, rattles, silver particles, or plasmonic hot spots, as described in Liu, N. et al. "Three-dimensional photonic metamaterials at optical frequencies" Nat. Mater. 7, 31-37 (2008); Khalavka, Y., Becker, J. & Sönnichsen, C. "Synthesis of Rod-Shaped Gold Nanorattles with Improved Plasmon Sensitivity and Catalytic Activity" J. Am. Chem. Soc. 131, 1871-1875 (2009); Jakab, A. et al. "Highly Sensitive Plasmonic Silver Nanorods" ACS Nano 5, 6880-6885 (2011) and Acimovic, S. S., Kreuzer, M. P., Gonzalez, M. U. & Quidant, R. "Plasmon Near-Field Coupling in Metal Dimers as a Step toward Single-Molecule Sensing" ACS Nano 3, 1231-1237 (2009).

The dramatic improvement in signal-to-noise ratio for plasmon sensors obtained by the invention for the goal of single molecule detection can also be used to improve other plasmonic sensors, e.g. plasmon rulers (see Liu, N., Hentschel, M., Weiss, T., Alivisatos, A. P. & Giessen, H. Three-Dimensional Plasmon Rulers. Science 332, 1407-1410 (2011)), or hydrogen sensors (see Liu, N., Tang, M. L., Hentschel, M., Giessen, H. & Alivisatos, A. P. Nanoantenna-enhanced gas sensing in a single tailored nanofocus. Nat. Mater. 10, 631-636 (2011)).

Individual molecule detection with individual plasmonic nanoparticles is not simply a dramatic downscaling of current sensors but a qualitative new step as it resolves molecular dynamics. With the invention, one can monitor desorption and adsorption processes in real time on a single molecule basis including conformational protein dynamics. By comparing different events, the influence of different regions of a protein and the protein folding dynamics can be evaluated, as well as conformational changes in a single bound protein. Additionally, the inventors demonstrate its suitability for observing equilibrium coverage fluctuations. These fluctuations hold information about binding kinetics and non-equilibrium thermodynamics. Single plasmon biosensors are therefore a new tool to study fundamental phenomena on the molecular level with impact for understanding of biological processes and the thermodynamics of small systems.

## Claims

1. A apparatus for sensing the presence and/or the behaviour of individual target molecules in a sample, comprising
- a plasmonic nanoparticle (6) which is exposed to the sample containing target molecules;
- a light source (1) illuminating the plasmonic nanoparticle (6) in a dark-field geometry;
- an optical device (10, 11, 12) for gathering and spectrally analysing the light scattered from the nanoparticle (6);
**characterized in that** the light source is a white light laser (1).

2. The apparatus of claim 1, **characterized in that** the optical device comprises an objective (10), a spectrometer (11) and an amplified charged-coupled device (CCD) camera (12), in particular an electron-multiplying CCD (EMCCD) camera.

3. The apparatus of any one of the preceding claims, **characterized in that** the nanoparticle (6) is rod-shaped having a width of 20 - 45 nm and an aspect ratio of above 2, preferably between 3 and 4.

4. The apparatus of any one of the preceding claims, **characterized in that** the nanoparticle (6) is immobilized in a micro-capillary, through which a liquid sample can be pumped.

5. The apparatus of any one of the preceding claims, **characterized in that** the dark-field geometry is achieved by disposing the plasmonic nanoparticle (6) close to a surface of a prism (3), which is illuminated by the light source (1) in total internal reflection geometry, such that the plasmonic nanoparticle (6) is reached by an evanescent light wave, wherein the prism is preferably a half-cylinder made of glass.

6. The apparatus of any one of the preceding claims, **characterized in that** the apparatus is capable of determining the plasmon resonance frequency with an accuracy of 0.003-0.2 nm, preferably 0.01-0.05 nm, most preferred about 0.03 nm.

7. A method of sensing the presence and/or the behaviour of individual target molecules in a sample, comprising the steps of:
- exposing a plasmonic nanoparticle (6) to the sample containing the target molecules;
- illuminating the plasmonic nanoparticle (6) by means of dark-field illumination;
- gathering the light scattered from the nanoparticle, spectrally investigating it and analysing the spectrum to detect a shift in plasmon resonance frequency caused by the presence and/or the behaviour of an individual target molecule;
**characterised in that** the plasmonic nanoparticle (6) is illuminated by a white light laser.

8. The method of claim 7, **characterised in that** it is carried out with the apparatus of any one of claims 1 to 6.

9. The method of any one of claims 7 to 8, **characterized in that** a mathematical function is fitted to the plasmon frequency spectrum, allowing the detection of shifts in plasmon resonance wavelength at an accuracy of 0.003-0.2 nm, preferably 0.01-0.05 nm.

10. The method of any one of claims 7 to 9, **characterised in that** the plasmon resonance frequency is measured at a time resolution between 50µs and 500ms, preferably between 1-100ms, most preferred between 10-50ms.

11. The method of any one of claims 7 to 10, **characterised in that** the shift in plasmon resonance frequency during one binding event, recorded at the time resolution according to claim 10 and averaged over several binding events, allows determination of the time scale of the binding event and/or the determination of a conformational change in the target molecule while it is bound or in close proximity to the nanoparticle.

12. The method of any one of claims 7 to 11, wherein the target molecule is a biological molecule having a size of 6-100nm, preferably 10-50nm, and in particular a polypeptide or protein having a molar weight between 1 and 3.000 kDa, preferably between 50 kDa and 500 kDa.

13. The method of any one of claims 7 to 12, wherein one or several capture molecules interacting specifically with the target molecules are attached to the nanoparticle, and wherein the measured shift in plasmon resonance frequency recorded at the time resolution of claim 10 is used for extracting a kinetic constant of an interaction between a target molecule and a capture molecule.

14. The method of one of claims 7 to 12, **characterised in that** the plasmonic nanoparticle surface is chemically modified (functionalized) to specifically attract a given target molecule.

15. Use of a plasmonic nanoparticle in a method according to one of claims 7 to 14, wherein the material, size, coating and/or shape of the nanoparticle is tailored to the target molecule.

16. Use of the method of any one of claims 7 to 14 for measuring equilibrium coverage fluctuations, in particular for extracting binding constants and/or binding kinetics for the interaction of the target molecule with a plasmonic nanoparticle from equilibrium coverage fluctuation.
